# EUROPEAN PATENT APPLICATION

(11) **EP 3 982 123 A1**
(43) Date of publication of application: **13.04.2022**
(21) Application number: 20382889.2
(22) Date of filing: 08.10.2020
(51) Int. Cl.: G01N 33/68

(54) **MARKERS AND THEIR USE IN BRAIN INJURY**

(71) Applicant: Fundació Hospital Universitari Vall d'Hebron - Institut de Recerca, 08035 Barcelona (ES); Université de Genève, 1211 Genève 4 (CH)
(72) Inventor: MONTANER VILLALONGA, Joan, 08037 Barcelona (ES); SANCHEZ, Jean-Charles, 1225 Chêne-Bourg (CH)
(74) Representative: ZBM Patents - Zea, Barlocci & Markvardsen

(57) **Abstract**

The invention relates to a combination of biomarkers and their use in brain injury or mild traumatic brain injury (mTBI) detection. The invention also provides an in vitro prospective method for determining a recovery outcome in one or more samples of a subject having experienced traumatic brain injury (TBI). Particular kits and means for carrying out the methods are also disclosed.

## Description

The invention relates to biomarkers and novel biomarkers, their use in diagnostics and prognosis of brain injury or brain related injuries, in particular mild traumatic brain injury (mTBI), and methods as well as devices (kits) for the detection of same in an individual.

### Background Art

Brain injuries have a high incidence worldwide. In particular mild traumatic brain injury (mTBI) has a significant incidence in the world and is responsible for high health cost. In contrast to severe TBI, mTBI related brain damage is not obvious to detect and thus usually a computer tomography (CT) scan is performed before significant brain injury can be ruled in or out.

So far, it is still a challenge identifying which patients with a number of neurological injuries and in particular a mild traumatic brain injury (mTBI), can be safely sent home. Computer tomography (CT) scan is thus the main tool today to detect a cerebral lesion in these patients. However, many of the scans are negative and cost-intense. Therefore, in any clinical decision rules for mild TBI, defined as presenting with a Glasgow Coma Score (GCS) (Jennet and Teasdale, check, 1978) of 13-15, rapid and reliable identification of patients with intracranial lesions is critical to avoid post-traumatic complications and minimize secondary brain damages. Several studies aimed to first screen all mild TBI-patients with a simple blood test to reduce the number of unnecessary CT scans and discharge patients faster have been reported. In the last years, especially S100B was extensively investigated as potential promising marker for mTBI and is highly promoted by companies. Nevertheless, its clinical utility remains controversial. In mild TBI adults, S100B below a cut-off of 0.10 pg/L was described to allow a maximal reduction of only 30% in CT scans. S100B failed to be a relevant prognostic marker for paediatric TBI patients, estimated only as adjunct in determining children with low-risk TBI.

A mild traumatic brain injury (mTBI), also called concussion, minor head trauma, and minor brain/head injury, is a type of closed head injury, in which the skull and dura mater remain intact, and it is defined as the result of a blunt trauma or acceleration/deceleration forces causing a brief change in mental status (confusion, disorientation or loss of memory) or loss of consciousness for less than 30 minutes. Usually, loss of consciousness is very brief and ranges between a few seconds to minutes. Mild TBI remains the biggest percentage of all closed head, brain injury cases admitted to the hospitals. Mild TBI are the leading cause of death in children under 4 years old and the most common cause of physical disability and cognitive impairment in young people. Currently, the primary criterion for evaluating patients with TBI in clinical setting is the Glasgow Coma Scale (GCS), which assesses the level of consciousness following TBI. A mild traumatic brain injury is most likely to be diagnosed only when there is a change in the mental status at the time of injury or hospital admission (the person is dazed, confused, or loses consciousness, GCS score 13-15). In US 10% of head injury patients are classified at admission as having severe (GSC below 8), 10% as moderate (GCS 9-12), and remaining 80% as mild TBI (GCS 13-15) (Narayan RK, Michel ME, et al, J Neurotrauma., 2002). Similar proportions are indicated by World Health Organization in Europe, that estimated 70 to 90% of treated head injuries are classified to present as mild (Cassidy JD et al, 2004, Journal of Rehabilitation Medicine). It remains a public health problem as 10% of patients with mTBI can suffer long-term disabilities such as headache, fatigue, difficulty thinking, memory problems, attention deficits, mood swings, sleep disorders, frustration and even epileptic events. Due to the complicated etiology it remains challenging to identify which patients with mTBI can be safely sent home without the need for treatment intervention. Currently to counter-act possible post-traumatic complications and secondary brain damages mTBI patients are further diagnosed with tools such as computerized tomography (CT) scans and magnetic resonance imaging (where available). In the group of patients with mTBI only 3 to 19% present with an abnormal CT result revealing an acute intracranial lesion in patients. The other 80%+ of these scans show normal head CT, indicating no complications from injury, and as such are not cost effective and are time-consuming for both patient and medical staff.

The use of biomarkers has been proposed as a means to reduce the amount of unnecessary CT scans and for use in decentralized sites where access to CT equipment is absent. However so far no biomarker-assay is available which gives test results capable of properly classifying the majority of patients and therefore useful in serial screening.

As described the method of choice today is a CT scan due to the insufficient reliability and high percentage of false negative results with known biomarkers for TBI detection. One such known biomarker is S100B.

In an injury like mTBI one cannot risk a significant percentage of false negatives in view of the detrimental consequences if a patient exhibiting mTBI would be allowed to leave the hospital and suffer serious complications, or even death thereafter due to a wrong diagnosis. Thus, the cut-offs defined for such tests need to be biased towards very high specificity (close to100%) which can result in a very low sensitivity in consequence. This limitation has made the known individual biomarkers for mTBI not feasible for routine diagnostics in a clinical setup.

In view of the cost pressure in healthcare and the high cost of a CT scan it is highly desirable and a long felt need to find alternative, reliable and cost-effective routes of classifying a potential mTBI patient.

Thus one object underlying the present application is to provide for alternative or new feasible biomarkers for the detection or/and classifying of any brain related traumatic state and in particular for mTBI, and for assays and devices useful and reliable therefore and in mTBI diagnostics which can be used in a clinical or non-clinical context, or to improve known approaches to neurological or mTBI screening and analysis.

Another need in the field of TBI management is the prediction of the outcome of those patients that suffered from a brain injury, in particular TBI, because these subjects as previously indicated may suffer from certain after discharge complications, such as cognitive disorders, behaviour disorders, emotional or physical impairments. All these may challenge the life quality of the patients in terms of having difficulties to perform daily routines and to work or have a social life. Although there are some markers that have been suggested as outcome indicators to increase clinical prediction, nowadays they have not provided the proper prediction capacities and are not suitable for clinical use. There is thus a need to provide reliable predictive biomarkers testable in isolated samples of patients to predict the most probable outcome of a patient that suffered from a brain injury, in particular a TBI, and even more in particular a mTBI.

### Summary of Invention

Inventors have surprisingly found that certain proteins in isolated biofluid samples are differentially detectable in patients (subjects) that have suffered TBI and that have intracranial lesions, even though said lesions are not physically or apparently evident at the first sight.

These proteins allow, individually and in combination, high specificity values around 30-50% individually, and around 50-70% of specificity in combination, for a fixed sensitivity of 95-100%. This means that patients that suffered from a TBI, and in particular mild TBI, (i.e. concussions) and having intracranial lesions usually detectable by means of brain image techniques (CT, MRI), can now be detected in a reliable way using a simple test from a sample (i.e. blood, plasma, serum, etc.) of the subject. The test provides a high probability of detecting all those TBI subjects that would result positive for intracranial lesion by means of an image technique, and safely classify a high percentage of patients that would result negative for an intracranial lesion by brain imaging.

The performance of the test allows selecting TBI patients classified as having an intracranial lesion for a subsequent brain imaging to value the lesion and/or to decide or recommend starting any therapeutic treatment or intervention.

This finding is surprising because, as previously indicated, the most promising marker to date, S100B levels in serum, allowed a maximal reduction of only 30% in CT scans. With the new proposed ones, the percentage of avoidable scans is increased without compromising the health of the patient that can be safely sent home.

Thus, a first aspect of the invention is an in vitro method for selecting a patient suffering traumatic brain injury (TBI) for a brain image technique and/or for a subsequent treatment intervention, comprising determining the level of expression of one or more of the proteins selected from the group consisting of FGFR1, AQP4, BCAN and A-FABP in an isolated sample of said patient.

FGFR1 stands for fibroblast growth factor receptor 1 (FGFR1).

BCAN stands for Brevican Core Protein.

AQP4 stands for Aquaporin-4.

A-FABP stands for fatty acid-binding protein in adipocytes.

This first aspect is to be interpreted, in general terms, as a method for screening for traumatic brain injury (TBI) comprising the steps of using an isolated sample of a subject (synonymous of patient) and detecting in such sample the level (i.e. level of expression) of one or more of the proteins selected from the group consisting of FGFR1, AQP4, BCAN and A-FABP.

As will be depicted in examples below, with only the determining of the levels of FGFR1, for a fixed 100% sensitivity (to catch all subjects with real positive CT, thus true positives for intracranial lesion), a specificity around 50% (i.e. 48.72 % in the assayed cohort) could be achieved. This specificity was increased if FGFR1 levels were combined with the levels of A-FABP (61.5 %), BCAN (53.8 %) and AQP4 (64.1 %).

Similar specificity values for a fixed sensitivity of 100% could be observed with the other three markers (i.e. levels of proteins), individually or in combination (2-marker panels).

Moreover, all these proteins allowed specificity values around 70-75 % for a sensitivity of 100 % if any of them were also combined with other proteins detected in samples of the subjects.

A second aspect of the invention relates to an in vitro method for screening for TBI patients with intracranial lesion, comprising determining the level of one or more of the proteins selected from the group consisting of FGFR1, AQP4, BCAN and A-FABP in an isolated sample of said patient.

Advantageously, the inventors could also determine that these same four proteins do also provide relevant information regarding the most probable recovery outcome of a subject with TBI. In a third aspect, the invention proposes also an in vitro prospective method for determining a recovery outcome in an isolated sample of a subject having experienced TBI, comprising determining the level of one or more of the proteins selected from the group consisting of FGFR1, AQP4, BCAN and A-FABP.

The invention provides the unexpected advantage that a brain injury and the particular medical complications as described herein can be identified and screened for not only in a fully equipped hospital but by use of a simple test device (i.e. kit) anywhere and without the use of qualified medical personnel. This is so, because the detection limit of these proteins in the isolated samples is in the order of nanograms per mililiter (ng/ml), which facilitates implementation of the test in a device or kit. This is of particular relevance, because previously proposed markers for TBI (such as the levels of GFAP) are in the order of picograms per milliliter, making difficult their implementation in test devices.

Invention also relates, in yet another aspect, to a kit comprising reagent means for detecting the level of two or more of the proteins selected from FGFR1, AQP4, BCAN and A-FABP.

In yet another aspect, the invention aims also the use of means for detecting the presence of any of FGFR1, AQP4, BCAN and A-FABP in a test sample of a subject suffering TBI, said means being selected from the group consisting of immunoassays, protein migration, chromatography, mass spectrometry, turbidimetry, nephelometry and polymerase chain reaction (PCR), for carrying out any of the methods as defined in previous aspects.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

"Brain injury" is any state of a patient or individual which is the cause of sudden impact on the head or the individual. A particular brain injury is TBI or mTBI.

"TBI" in the sense of the invention is any brain injury caused by a traumatic incident as described above with reference to the prior art. A traumatic brain injury (TBI), also known as an intracranial injury, is an injury to the brain caused by an external force. TBI can be classified based on severity (ranging from mild traumatic brain injury [mTBI/concussion] to severe traumatic brain injury), mechanism (closed or penetrating head injury), or other features (e.g., occurring in a specific location or over a widespread area)

"Identification" or "identify" or "classify" in the sense of the invention is the analysis of a sample of an individual to assess whether the individual has a brain injury, that is intracranial lesions, particularly in TBI subjects or mTBI subjects; the identification of e.g. TBI and mTBI (that is, of lesions in brain) can be verified by use of a CT scan or MRI analysis.

The term "patient" (or subject, as synonymous), as used herein, refers to any subject which show one or more signs or symptoms typically associated with TBI, and in particular mTBI. The term "patient", as used herein, refers also to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race. Preferably the patient suffers stroke.

The term "selecting a patient or a TBI subject for an image technique and/or subsequent treatment.", as used herein, relates to the identification of a patient that after the analysis of the proteins levels in the isolated sample will be further submitted to imaging of the brain to determine the intracranial lesion type and degree. It also relates to the identification of a patient, that as a result of the test sample is determined as having intracranial lesion and requires of the application of therapeutic interventions to avoid complications.

The term "level of expression of one or more proteins" or "level of one or more proteins in a sample" relates to the amount of the protein expressed as a concentration, usually the weight of the protein in a volume of sample.

An "Assay" or "test" in the sense of the invention is any method generally known in the art to detect TBI or mTBI like ELISA or any other standard methods for detection of protein levels or expression of genes (biomarkers), as will be explained in more detail below.

"Device" or "kit" in the sense of the invention is a combination of the biomarkers (levels of proteins of interest in a sample) or panel of biomarkers according to the invention that can be used to perform an assay for TBI or mTBI detection with intracranial lesion. Examples are carrier plates, test stripes, biochip arrays, or the like known in the art including the reagent means to detecting the presence and level of the proteins of interest. More detailed explanations are listed below.

"Marker" or "biomarker" or "molecular marker" or "molecular biomarker" or "protein level" or "gene expression level" (all used interchangeable) in the sense of the invention, is any useful biomarker to detect in a sample of preferably blood, plasma, saliva, tears, CSF or urine, a brain injury, preferably TBI, more in particular mTBI with intracranial lesion. The markers (i.e. protein levels) are used in a suitable assay setup wherein in particular the sensitivity is set to 100 %, or from 95 % to 100 %, and the specificity is in particular more than 30% or more than 40%, even more in particular more than 50%, more than 55%, more than 58%, 60%, 70% or even 75%.

A marker "panel" in the sense of the invention is a combination of at least two biomarkers (the levels of two proteins in the sample), in particular two or three or four markers, used in combination in a suitable setup or device.

"Sensitivity" in the sense of the invention refers to the assay result of true positives in the analysis of TBI or mTBI with intracranial lesion. Preferably the sensitivity in the analysis according to the invention is set to 95% to 100%, or 100% (i.e. no false negative diagnoses).

"Specificity" in the sense of the invention is the so-called true negative rate in an assay to identify TBI or mTBI with intracranial lesion. The specificity is preferably targeted to be at least 30% or 40%, 50% and preferably higher, e.g. 58%, 60%, 65%, 70%.

A "sample" or "specimen" in the sense of the invention is any fluid or tissue useful for performing an assay or detection method to identify TBI, preferably mTBI. Preferably the sample is a blood, plasma or urine sample taken from an individual. The sample is treated according to generally known procedures to keep or make them feasible for the marker analysis according to the invention.

A"predictive" or "prospective" method in the sense of the invention is a method useful in providing information on a future development of a patient or individuum or subject who has been subject to a certain experienced or an impact or a shock or a traumatic incident, e.g. a brain injury, and in particular the recovery of said subject from said brain injury after a defined time span; said time span may be 1, 2, 3, 4, 5, or 6 months, or 1 or 2 years.

As will be apparent from the experimental part describing the invention, the invention has the advantage that it achieves very reliable test results. Accordingly, in preferred embodiments it provides for a method, kit or assay wherein the sensitivity is more than 95%, preferably 100%, and the specificity is more than 50%, preferably more than 55%, more preferably more than 60%, and more preferably more than 70%.

As indicated in the summary, present invention relates in a first aspect to an *in vitro* method for selecting a patient suffering traumatic brain injury (TBI) for a brain image technique and/or for a subsequent treatment intervention, comprising determining the level of one or more of the proteins selected from the group consisting of FGFR1, AQP4, BCAN and A-FABP in an isolated sample of said patient.

In a particular embodiment of the first aspect the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

Unexpectedly and surprisingly the inventors could show that by a combination of at least the levels of two proteins of FGFR1, BCAN, AQP4 and A-FABP, a reliable and easy to use method can be provided to reliably analyse a specimen and thereby rule out brain injury complications in an individual characterized by mTBI and thereby to avoid costly CT scans or even transportation to a centre capable of performing such.

It was not predictable that the markers of the invention and the particular selection of certain markers or/and the combination of certain markers could be applied to provide for a reliable and specific method to assay for the brain injuries or disorders or diseases or medical complications as described herein and particularly TBI and mTBI. The invention will have not only a positive impact on cost in such analysis and specifically mTBI analysis but represents also an easy to use and fast method in this medical area.

In even a more particular embodiment of the in vitro method for selecting a patient suffering TBI according to the first aspect, the method comprises determining the level of expression of at least one set of proteins selected from the group consisting of: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B; BCAN and FGFR1; BCAN and H-FABP; BCAN and IL-10; BCAN and S100B; FGFR1 and H-FABP; FGFR1 and GFAP; FGFR1 and IL-10; and FGFR1 and S100B. Thus, the method comprises determining the level of the two different proteins of one or more of these sets.

In also another particular embodiment of the first aspect, the levels of three of FGFR1, AQP4, BCAN, and A-FABP, or of the four are determined. In another also particular embodiment, the method comprises determining the level of at least one or more of FGFR1, AQP4, BCAN, and A-FABP; and at least one or more of H-FABP, GFAP, IL-10, and S100B. In even another particular embodiment, the method comprises determining the level of expression of the eight proteins FGFR1, AQP4, BCAN, A-FABP; H-FABP, GFAP, IL-10, and S100B.

This is the first time any of the levels of FGFR1, AQP4, BCAN and A-FABP are correlated with the capability of selecting a TBI subject for an image technique and/or subsequent treatment. In a particular embodiment, optionally in combination with any of the embodiments above or below, the level of the one or more proteins are compared with a corresponding reference value and the patient is selected for a brain image technique and/or for a subsequent treatment intervention when the level of FGFR1 and/or of BCAN is lower than a reference value (also named as a cut off or threshold value); and/or when the level of A-FABP and/or of AQP4 is higher than a reference value.

Indeed, the skilled person will understand that any step of comparing the levels of the proteins in the test sample (patient) with a reference may be performed in several ways but, the rationale is that the levels are compared with a corresponding reference value to determine if the patient is to be selected for a brain image technique and/or for a subsequent treatment intervention.

The term "reference" or "reference value" or "cut off" or "threshold", as used herein, relates to a predetermined criterion used as a reference for evaluating the values or data obtained from the samples collected from a subject. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group or based on a pool of samples including or excluding the sample to be tested. The skilled person in the art, making use of the general knowledge, can choose the subject or group of subjects more adequate for obtaining the reference value for each of the methods of the present invention. Methods for obtaining the reference value from the group of subjects selected are well-known in the state of the art (Burtis C. A. et al., 2008, Chapter 14, section "Statistical Treatment of Reference Values") In a particular case the "reference value" is a cut-off value or threshold defined, for example, by means of a conventional ROC analysis (Receiver Operating Characteristic analysis). Reference values have been determined for the biomarkers of the invention. The reference value for each of FGFR1, AQP4, BCAN and A-FABP, and other proteins in this description may be from a lower and an upper value as will be disclosed in view of examples below. Range of values of each biomarker (protein levels) and particular combinations of the values of the different biomarkers provide for correct classification of subjects with high sensitivity and specificity.

Thus, in another example of the method of the first aspect, the level of the one or more proteins are compared with a corresponding reference value and the patient is selected for a brain image technique and/or for a subsequent treatment intervention, when the level of one or more of the listed proteins are within a reference range level of a patient with intracranial lesion, or alternatively such levels of the test sample match with a reference value/range indicating proper selection of imaging/treatment.

The levels of a biomarker (in this invention any of FGFR1, BCAN, AQP4 and A-FABP, as well as the level of other proteins in combination with any of them) are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more higher than the reference value.

Likewise, in the context of the present invention, the level of a biomarker is reduced when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than the reference value.

In another particular embodiment of the first aspect, the brain image technique is tomography computer tomography (CT) and/or magnetic resonance imaging (MRI).

Also, in another particular embodiment of the first aspect, the treatment intervention is selected from surgery, in particular for relieving pressure in the skull, and/or for removing clotted blood and/or for repairing skull fractures; medication with neuroprotectants, and/or with anticoagulants and/or with anticonvulsants; and/or physical and/or psycological rehabilitation.

Thus, in another particular embodiment of the first aspect, the brain image technique is tomography computer tomography (CT) and/or magnetic resonance imaging (MRI); and the treatment intervention is selected from surgery for relieving pressure in the skull, and/or surgery for removing clotted blood and/or surgery for repairing skull fractures, medication with neuroprotectants, and/or medication with anticoagulants and/or medication with anticonvulsants, and/or physical and/or psycological rehabilitation.

As a second aspect the invention relates to an in vitro method for screening for TBI patients with intracranial lesion, comprising determining the level of one or more of the proteins selected from the group consisting of FGFR1, AQP4, BCAN and A-FABP in an isolated sample of said patient.

The term "intracranial lesion" in the context of TBI includes any damage to the brain tissue due to the traumatic incident. In the particular context of mTBI (closed-head injuries) the lesions or injuries include: concussion (temporary dysfunction of normal brain function), intracranial hematomas (i.e. rupture of blood vessel causing pool of blood around the around the brain, called subdural hematoma, or between the brain and the skull, called epidural hematoma), and cerebral contusions (bruise to the brain tissue as a result of trauma).

In the context of mTBI these possible brain injuries are not apparent in most of the cases until CT or MRI is performed. Their detection is of vital importance to counter-act possible post-traumatic complications and secondary brain damages in those percentage of TBI patients (10%) with mTBI that can suffer long-term disabilities such as headache, fatigue, difficulty thinking, memory problems, attention deficits, mood swings, sleep disorders, frustration and even epileptic events

As indicated for the method of the first aspect, in a particular embodiment of the in vitro method for screening for TBI patients with intracranial lesion according to the second aspect, the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

In another particular embodiment of the second aspect, the level of the proteins (or markers) that are determined is a binary combination selected from one or more of the following sets of proteins: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B; BCAN and FGFR1; BCAN and H-FABP; BCAN and IL-10; BCAN and S100B; FGFR1 and H-FABP; FGFR1 and GFAP; FGFR1 and IL-10; and FGFR1 and S100B.

In also another particular embodiment of the second aspect, the levels of three of FGFR1, AQP4, BCAN, and A-FABP, or of the four are determined. In another also particular embodiment, the method comprises determining the level of at least one or more of FGFR1, AQP4, BCAN, and A-FABP; and at least one or more of H-FABP, GFAP, IL-10, and S100B. In even another particular embodiment, the method comprises determining the level of expression of the eight proteins FGFR1, AQP4, BCAN, A-FABP; H-FABP, GFAP, IL-10, and S100B.

All the particular embodiments, regarding the comparison with reference values of the determined levels of the proteins in the isolated sample indicated for the first aspect, do also apply to this second aspect.

In another particular embodiment of any of the in vitro methods of the first and the second aspect, the method further comprises determining one or more clinical parameters, and/or combining the levels of the one or more proteins determined with gender and/or age of the patient.

In a more particular embodiment, the above-referred clinical parameters are selected from the group consisting of blood pressure, including systolic blood pressure and/or diastolic blood pressure, scores from systematic assessment tools of TBI (Glasgow Coma Score), and combinations thereof.

In another particular embodiment of any of the in vitro methods of the first and the second aspect, the isolated biological sample is a biofluid selected from the group consisting of blood, serum, plasma, urine, saliva, lachrymal fluid, cerebrospinal fluid, and combinations thereof. In a more particular embodiment, it is serum.

As previously indicated, the methods of the first and second aspect are particularly applicable to a patient suffering mild traumatic brain injury (mTBI).

Interestingly, inventors could prove that the levels of above-indicated four proteins (FGFR1, AQP4, BCAN and A-FABP), individually, or in combination of two, three or the four, as well as any of them in combination with other markers, allow predicting the recovery outcome of a patient that has suffered a TBI, in particular a mild TBI. Thus, in another particular embodiment of the methods of the first and second aspects, these methods further comprise determining a recovery outcome by comparing the level obtained for the one or more of the proteins with a corresponding reference value to determine the recovery outcome.

As will be illustrated in examples below, and for the third aspect of the invention, this recovery outcome is assessed by means of the Glasgow Outcome Scale Extended (GOSE) score, according to which a GOSE <8 indicates an incomplete recovery, and a GOSE = 8 indicates a complete recovery.

The Glasgow Outcome Score (GOS) is a scale of patients with brain injuries, such as cerebral traumas that groups victims by the objective degree of recovery (Jennett, B; Bond, M (Mar 1, 1975). *"*Assessment of outcome after severe brain damage". Lancet. 1 (7905): 480-484*.).* The Glasgow Outcome Scale Extended (GOSE) is an expanded version of the scale, including eight categories: 1- death; 2- vegetative state; 3- Lower severe disability; 4- upper severe disability; 5- lower moderate disability; 6- Upper moderate disability - some disability but can potentially return to some form of employment; 7- Lower good recovery-minor physical or mental defect; and 8- upper good recovery or full recovery.

Another aspect of the invention, the third aspect, is an in vitro prospective method for determining a recovery outcome in an isolated sample of a subject having experienced traumatic brain injury (TBI), comprising determining the level of one or more of the proteins selected from the group consisting of FGFR1, AQP4, BCAN and A-FABP.

In a particular embodiment of the third aspect, the in vitro prospective method for determining a recovery outcome in an isolated sample of a subject having experienced traumatic brain injury (TBI), comprises the steps of :
(a) determining the level of one or more of the proteins selected from the group consisting of FGFR1, AQP4, BCAN and A-FABP; and
(b) comparing each level obtained under step a) with a corresponding reference value to determine the recovery outcome.

In another particular embodiment of this prospective method of the third aspect, the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

In even a more particular embodiment of the of this prospective method of the third aspect, the method comprises determining the level of expression of at least one set of proteins selected from the group consisting of: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B;BCAN and FGFR1; BCAN and H-FABP; BCAN and IL-10; BCAN and S100B; FGFR1 and H-FABP; FGFR1 and GFAP; FGFR1 and IL-10; and FGFR1 and S100B. Thus, the method comprises determining the level of the two different proteins of one or more of these sets.

In also another particular embodiment of the prospective method for determining a recovery outcome, the levels of three of FGFR1, AQP4, BCAN, and A-FABP, or of the four are determined. In another also particular embodiment, the method comprises determining the level of at least one or more of FGFR1, AQP4, BCAN, and A-FABP; and at least one or more of H-FABP, GFAP, IL-10, and S100B. In even another particular embodiment, the method comprises determining the level of expression of the eight proteins FGFR1, AQP4, BCAN, A-FABP; H-FABP, GFAP, IL-10, and S100B.

This is the first time any of the levels of FGFR1, AQP4, BCAN and A-FABP are correlated with the capability of determining a recovery outcome in an isolated sample of a subject having experienced traumatic brain injury (TBI). In a particular embodiment, optionally in combination with any of the embodiments above or below, the recovery outcome is assessed by means of the Glasgow Outcome Scale Extended (GOSE) score.

In yet another more particular embodiment of the prospective method, the level of the one or more proteins are compared with a corresponding reference value, and the recovery outcome corresponds to a GOSE <8, or an incomplete recovery 3-months after the trauma, if the level of FGFR1 and/or of BCAN is lower than a reference value; and/or when the level of A-FABP and/or of AQP4 is higher than a reference value. On the alternative, a recovery outcome corresponding to a GOSE = 8 is determined if level of FGFR1 and/or of BCAN is higher than a reference value; and/or when the level of A-FABP and/or of AQP4 is lower than a reference value.

Examples below will illustrate that for a fixed sensitivity of 95%-100 % (to catch all TBI patients with a most probable recovery outcome corresponding, indeed, to an incomplete recovery; GOSE<8), specificity values ranged from 20% to 50% when one of the four proteins FGFR1, AQP4, BCAN and A-FABP were used. Specificity increased with combinations (two-marker panels) of the levels of these proteins, attaining values from 60 % to 76 % of specificity.

The prediction of the most probable outcome in the patients that experienced a TBI, in particular a mild TBI with an easy to perform test, will help to take certain medical decisions as soon as possible to reduce the bad progression (if possible) in patients with poor outcome.

All the particular embodiments indicated for the first aspect and second aspects, regarding the comparison with reference values of the determined levels of the proteins in the isolated sample, as well as regarding the possible combination with one or more clinical parameters, and/or the combination of the levels of the one or more proteins determined with gender and/or age of the patient, as well as regarding the type of biological sample, do also apply to this prospective method for determining a recovery outcome.

In some particular embodiments of any of the methods of the first, second and third aspects, the method further comprises the step of selecting a therapy for the patient that has been classified as having intracraneal lesions, or that has been selected for intervention, or thas has been classified as having a most probable recovery outcome with a GOSE < 8.

Thus, after the classification of the TBI patient for brain imaging and/or for subsequent therapy, or after the classification of being a TBI patient with intracranial lesions, or after the determination of the outcome, the methods comprise a step of recommending a therapy and/or treating said patient. In a moreparticular embodiment, such therapy includes surgery for relieving pressure in the skull, and/or surgery for removing clotted blood and/or surgery for repairing skull fractures, medication with neuroprotectants, and/or medication with anticoagulants and/or medication with anticonvulsants, and/or physical and/or psycological rehabilitation.

This particular embodiment could be drafted as a method of treating a patient suffering TBI, in particular mild TBI, said method comprising carrying out the in vitro method of any of the first, second and third aspects, and treating the patient with surgery for relieving pressure in the skull, and/or surgery for removing clotted blood and/or surgery for repairing skull fractures, medication with neuroprotectants, and/or medication with anticoagulants and/or medication with anticonvulsants, and/or physical and/or psycological rehabilitation.

When in present invention FGFR1 is referred, it relates to fibroblast growth factor receptor 1 (FGFR1), also known as basic fibroblast growth factor receptor 1, fms-related tyrosine kinase-2 / Pfeiffer syndrome, and CD331, is a receptor tyrosine kinase whose ligands are specific members of the fibroblast growth factor family. The complete sequence for human FGFR1 has, in particular, the UniProtKB accession number P11362 (May 1, 1991, version 3 of the sequence and version 179 of the database).

BCAN relates to Brevican Core Protein, a protein that is encoded by the *BCAN* gene. It is a member of the lectican protein family and localised to the surface of neurons in the brain. The complete sequence for human BCAN has, in particular, the UniProtKB accession number Q96GW7.

AQP4 relates to Aquaporin-4, a water channel protein encoded by the AQP4 gene in humans. In the CNS, AQP4 is the most prevalent aquaporin channel, specifically located at the perimicrovessel astrocyte foot processes, glia limitans, and ependyma. In addition, this channel is commonly found facilitating water movement near cerebrospinal fluid and vasculature. The complete sequence for human AQP4 has, in particular, the UniProtKB accession number P55087 (November 1, 1997, version 2 of the sequence and version 261 of the database).

A-FABP relates to the Fatty acid-binding protein in adipocytes, a lipid transport protein in adipocytes. The complete sequence for human A-FABP has, in particular, the UniProtKB accession number P15090 (January 23, 2007, version 3 of the sequence and version 201 of the database).

H-FABP, is fatty acid-binding protein in heart. The complete sequence for human A-FABP has, in particular, the UniProtKB accession number P05413 (January 23, 2007, version 4 of the sequence and version 196 of the database).

GFAP is refers to glial fibrillary acidic protein, an intermediate filament protein that is expressed by numerous cell types of the central nervous system. The complete human sequence for glial fibrillary acidic protein has, in particular, the UniProtKB accession number P14136 (January 1^{st}, 1991, version 1 of the sequence and version 2017 of the database).

IL-10 is interleukine-10. The complete human sequence for IL-10 has, in particular, the UniProtKB accession number P22301 (August 1, 1991, version 1 of the sequence and version 204 of the database).

S100B relates to protein S-100-B. The complete human sequence for S100B has, in particular, the UniProtKB accession number P04271 (January 23, 2007, version 2 of the sequence and version 211 of the database).

All these proteins do have homologues in other mammal species (cats, dogs, mouse, rats, etc.). The skilled man can retrieve the corresponding complete sequences in public databases.

As the person skilled in the art understands, the expression levels of these proteins can be determined by measuring the levels of mRNA encoded by the corresponding genes or by measuring the levels of proteins encoded by said genes, and the levels of variants thereof.

By way of a non-limiting illustration, the expression levels are determined by means of the quantification of the levels of mRNA encoded by said genes. The latter can be quantified by means of using conventional methods, for example, methods comprising the amplification of mRNA and the quantification of the amplification product of said mRNA, such as electrophoresis and staining, or alternatively, by means of Northern blot and the use of suitable probes, Northern blot and use of specific probes of the mRNA of the genes of interest or of their corresponding cDNA/cRNA, mapping with the SI nuclease, RT-PCR, hybridization, microarrays, etc. Similarly, the levels of the cDNA/cRNA corresponding to said mRNA encoded by the marker genes can also be quantified by means of using conventional techniques; in this event, the method of the invention includes a step of synthesis of the corresponding cDNA by means of reverse transcription (RT) of the corresponding mRNA followed by the synthesis (RNA polymerase) and amplification of the cRNA complementary to said cDNA. Conventional methods of quantifying the expression levels can be found in laboratory manuals.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts. Preferably, the control RNA is mRNA derived from housekeeping genes and which code for proteins which are constitutively expressed and carry out essential cellular functions. Preferred housekeeping genes for use in the present invention include 18-S ribosomal protein, β-2-microglobulin, ubiquitin, cyclophilin, GAPDH, PSMB4, tubulin and β-actin.

Alternatively, it is also possible to determine the expression levels of the marker genes by means of the determination of the expression levels of the proteins encoded by said genes, since if the expression of genes is increased, an increase of the amount of corresponding protein should occur and if the expression of genes is decreased, a decrease of the amount of corresponding protein should occur.

The determination of the expression levels of the proteins can be carried out by qualitative and/or quantitative tests selected from the group consisting of an immunological test, bioluminescence, fluorescence, chemiluminescence, electrochemistry and mass spectrometry. Particular tests that can be implemented in a point of care test format (POCT) are recommended to make easy and fast the determining of marker levels. In a particular embodiment, point of care tests include lateral flow tests, which allow detecting the presence (or absence) of a target analyte in liquid sample (matrix) without the need for specialized and costly equipment, though many lab-based applications exist that are supported by reading equipment.

Independently of the test format, particular quantitative tests are selected from the group consisting of an immunological test, bioluminescence, fluorescence, chemiluminescence, electrochemistry and mass spectrometry.

In one embodiment, the level of expression is determined by immunological techniques such as enzyme-linked immunosorbent assay (ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan, such as radioimmunoassay, as well as protein microarray formats, such as single molecular assay (SIMOA), Western Blot or immunofluorescence.

Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry, fluorometry, mass spectrometry or tandem mass tags (TMT). SIMOA is a type of assay more sensitive than an ELISA, since it uses arrays of femtoliter-sized reaction chambers, which are termed single-molecule arrays (SimoaTM) that can isolate and detect single enzyme molecules. Because the array volumes are approximately 2 billion times smaller than a conventional ELISA, a rapid build-up of fluorescent product is generated if a labeled protein is present. With diffusion defeated, this high local concentration of product can be readily observed. Only a single molecule is needed to reach the detection limit. Using the same reagents as a conventional ELISA, this method has been used to measure proteins in a variety of different matrices (serum, plasma, cerebrospinal fluid, urine, cell extracts, etc.) at femtomolar (fg/mL) concentrations, offering a roughly 1000-fold improvement in sensitivity.

On the other hand, the determination of the protein expression levels can be carried out by constructing a tissue microarray (TMA) containing the subject samples assembled, and determining the expression levels of the proteins by techniques well known in the state of the art.

In a preferred embodiment the determination of the levels of the markers are determined by immunological technique. In a more preferred embodiment, the immunological technique is ELISA.

When an immunological method is used, any antibody or reagent known to bind with high affinity to the target proteins can be used for detecting the amount of target proteins. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

As previously cited, the expression levels in an isolated sample of a patient of FGFR1, AQP4, BCAN, A-FABP, and other proteins listed in the methods, kits and systems of the invention, can be determined by measuring both the levels of protein, and the levels of variants thereof, such as fragments, isoforms, analogues and/or derivatives.

As known in the art the "similarity" between two proteins is determined by comparing the amino acid sequence and its conserved amino acid substitutes of one protein to a sequence of a second protein. The variants are defined to include polypeptide sequences different from the original sequence, preferably different from the original sequence in less than 40% of residues per segment concerned, more preferably different from the original sequence in less than 25% of residues per segment concerned, more preferably different from the original sequence in less than 10% of residues per segment concerned, more preferably different from the original sequence in only a few residues per segment concerned and, at the same time, sufficiently homologous to the original sequence to preserve functionality of the original sequence. Variants according to the present invention includes amino acid sequences that are at least 60%, 65%, 70%, 72%, 74%, 76%, 78%, 80%, 90%, or 95% similar or identical to the original amino acid sequence. The degree of identity between two proteins is determined using computer algorithms and methods that are widely known for the persons skilled in the art. The identity between two amino acid sequences is preferably determined by using the BLASTP algorithm [BLASTManual, Altschul, S., et al, NCBI NLM NIH Bethesda, Md. 20894, Altschul, S., et al, J. Mol. Biol. 215: 403-410 (1990)].

The proteins can be post-translationally modified. For example, post- translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

In another particular embodiments of the methods of the first, second and third aspects of the invention, they further comprise the steps of (i) collecting the information of the selection for imaging/therapy, presence of intracranial lesion and/or most probable recovery outcome, and (ii) saving the information in a data carrier.

In the sense of the invention a "data carrier" is to be understood as any means that contain meaningful information data for the selection, presence of intracranial lesion and/or most probable recovery outcome, such as paper. The carrier may also be any entity or device capable of carrying the said information. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the information data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

For the implementation of the method of any of the first, second and third aspects, in a particular embodiment when the level of at least two proteins are determined, a computer-implemented system operates, said system comprising:
(a) at least two databases comprising:
   (i) a first test result collected from a first diagnostic test for one of the proteins;
   (ii) a second test result collected from a second diagnostic test for a second protein different than the first diagnostic test;
   (iii) optionally, subsequent test result(s) collected from subsequent diagnostic test(s) different from the previous diagnostic test(s);
   (iv) optionally , secondary subject observations or measurements;
   (v) one or more diagnostic cut-offs or reference values associated with the first diagnostic test, with the second diagnostic test, with subsequent diagnostic tests, and with the subject observations or measurements, wherein such cut-offs and reference values collectively integrate to assess probability of brain injury status, in particular of intracranial lesion.
(b) one or more processors operatively encoded to automatically:
   (i) apply an interpretation algorithm to generate a subject result coordinate based on the database of test results;
   (ii) optionally apply a second interpretation algorithm to generate a probability of error in the subject result coordinate.

As previously indicated, the invention also relates to kits comprising reagent means for detecting the level of the level of two or more of the proteins selected from FGFR1, AQP4, BCAN and A-FABP.

In a particular embodiment of the kits of the invention, they comprise reagent means for determining the levels of at least two different proteins selected from FGFR1, AQP4, BCAN and A-FABP, or two or more of these four proteins, and further reagent means for determining the levels of one or more additional proteins selected from H-FABP, GFAP, IL-10, and S100B.

In another particular embodiment of the kits of the invention, they comprise reagent means for determining the levels of the proteins of the sets selected from the group consisting of: A-FABP and AQP4; A-FABP and BCAN; A-FABP and FGFR1; AQP4 and BCAN; AQP4 and FGFR1;and BCAN and FGFR1; BCAN and H-FABP; BCAN and IL-10; BCAN and S100B; FGFR1 and H-FABP; FGFR1 and GFAP; FGFR1 and IL-10; and FGFR1 and S100B.

Other kits disclosed in this description comprise reagent means for determining the levels of the proteins of the sets selected from the group consisting of: A-FABP and H-FABP; A-FABP and GFAP; A-FABP and IL-10; A-FABP and S100B; AQP4 and H-FABP; AQP4 and GFAP; AQP4 and IL-10; and AQP4 and S100B; BCAN and H-FABP; BCAN and IL-10; BCAN and S100B; FGFR1 and H-FABP; FGFR1 and GFAP; FGFR1 and IL-10; and FGFR1 and S100B.

Particular kits, as used herein, refer to a product containing the different reagents (or reagent means) necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes.

Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally, or alternatively, the media can contain internet addresses that provide said instructions.

The reagent means (or simply reagents) of the kit include compounds that bind specifically to the marker proteins. Preferably, said compounds are antibodies, aptamers or fragments thereof.

In a preferred embodiment, the reagent is an antibody or fragment thereof. Thus, the reagent means are one or more antibodies that specifically recognize the proteins of interest (i.e. antibodies or fragments to for at least two of FGFR1, AQP4, BCAN and A-FABP, and optionally any additional proteins in combination with any of these four). The antibodies of the kit of the invention can be used according to techniques known in art for determining the protein expression levels, such as, for example, flow cytometry, Western blot, ELISA, RIA, competitive EIA, DAS-ELISA, techniques based on the use of biochips, protein microarrays, or assays of colloidal precipitation in reactive strips.

The antibodies can be fixed to a solid support such as a membrane, a plastic or a glass, optionally treated to facilitate the fixation of said antibodies to the support. Said solid support comprises, at least, a set of antibodies which specifically recognize the marker (i.e. the protein of interest), and which can be used for detecting the levels of expression of said marker.

Additionally, the kits of the invention comprise reagents for detecting a protein encoded by a constitutive gene. The availability of said additional reagents allows normalizing the measurements performed in different samples (for example, the sample to be analyzed and the control sample) to rule out that the differences in the expression of the biomarkers are due to a different quantity of total protein amount in the sample more than the real differences in the relative levels of expression. The constitutive genes in the present invention are genes that are always active or being transcribed constantly and which encode for proteins that are expressed constitutively and carry out essential cellular functions. Proteins that are expressed constitutively and can be used in the present invention include, without limitation, β-2-microglobulin (B2M), ubiquitin, 18-S ribosomal protein, cyclophilin, GAPDH, PSMB4, tubulin and actin.

In a preferred embodiment, the reagent means for assaying the levels of the different proteins comprise at least 10%, at least 20%, at least 30%, at least 40%, at least 50%, at least 60%, at least 70%, at least 80%, at least 90% or at least 100% of the total amount of reagents for assaying the protein levels in said kit. Thus, in the particular case of kits comprising reagents for assaying the levels of at least two of FGFR1, AQP4, BCAN and A-FABP, and optionally any additional proteins in combination with any of these four, the reagents specific for said proteins comprise at least 10%, at least 20%, at least 30%, at least 40%, at least 50%, at least 60%, at least 70%, at least 80%, at least 90% or at least 100% of the reagents (i.e antibodies) present in the kit. These kits are, thus, simplified kits including mainly the reagent means for detecting the levels of the two (or three) proteins.

In another embodiment, the kits of the invention are conceived as point of care tests. More they are in form of lateral flow tests.

In another particular embodiment the kit according to the invention comprises a support and one or more sample inlet ports for deposition of a biofluid sample, in particular whole blood; a reaction area comprising the means /reagents that bind specifically to the marker proteins, in particular antibodies; and wherein the sample inlet port is connected with the reaction area. In another more particular embodiment, the kit comprises as many sample inlet ports as markers (one, two or three) to be detected and corresponding reaction areas connected thereto. In another embodiment the kit comprises one single inlet import and as capillary tracks connecting to as many reactive areas, said capillary tracks conducting part of the sample to each corresponding connected reaction area. The kits comprising more than one reaction areas are multiplex kits.

This invention also discloses an in vitro method of identifying, in a sample taken from a subject suffering from TBI, in particular mTBI, the presence or absence, and if present the level of expression of one or more of the proteins selected from the group consisting of FGFR1, AQP4, BCAN and A-FABP.

It is also disclosed herein an in vitro method of detecting one or more of the proteins selected from the group consisting of FGFR1, AQP4, BCAN and A-FABP in an isolated sample of a patient of TBI, in particular of mTBI, the method comprising:
a. obtaining a sample from the patient;
b. conducting an assay to detect the level of FGFR1 in the sample; and/or
c. conducting an assay to detect the level of AQP4 in the sample; and/or
d. conducting an assay to detect the level of BCAN in the sample; and/or
e. conducting an assay to detect the level of A-FABP in the sample; and/or
wherein if more than one assay is conducted, they are performed sequentially in any order or simultaneously.

The detection of the levels in these assays is performed, as indicated by mean sof reagents that specifically recognize the proteins in the sample, such as an antibody.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### METHODOLOGY and STUDY DESIGN

Longitudinal, prospective study, including patients with mild TBI (Glasgow Coma Scale = 14-15) within the first 6 hours after trauma. Patients enrolled at the emergency department (ED) of Hospital Virgen del Rocío in Seville and biomarkers were measured at Vall d'Hebron Research Institute (VHIR) in Barcelona and Geneva University.

### STUDY PARTICIPANTS

### Inclusion criteria

- Age> 18 years
- Mild TBI within the first 6 hours after trauma, defined as Glasgow Coma Scale (GCS) of 14-15 points plus the presence of any of the following:
   ∘ Loss of consciousness shorter than 30 minutes, within 20 minutes after trauma.
   ∘ Post-traumatic amnesia shorter than 24 hours, within 30 minutes after trauma.

### Exclusion criteria

- Dementia or other pre-existing neurological conditions that difficult evaluation.
- Recent history (<1 month) of head trauma.
- Refusal to participate in the study or give informed consent.
- Evidence of intoxication with alcohol or other substances.

### STUDY PROCEDURES

Patients were identified and enrolled at hospital admission, at the time of ED arrival. After informed consent signature, the blood sample for testing of the expression levels of FGFR1, AQP4, BCAN, A-FABP, H-FABP, GFAP, IL-10, and S100B was obtained, before any brain CT scan. Two-8.5cc serum and two 6cc plasma-ETDA tubes were collected by venipuncture, together with the routine ED analyses. These tubes were centrifuged and aliquoted in 500-microliter microtubes. The samples were locally stored frozen at -20°C, and then transferred to the study biobank, at the Neurovascular Research Laboratory of VHIR, in Barcelona, to be stored frozen at -80° until analysis. Samples were preserved as a sample collection, registered with the Instituto de Salud Carlos III (code C.0004519). The levels in the samples of FGFR1, AQP4, BCAN, A-FABP, H-FABP, GFAP, IL-10, and S100B were analyzed with commercially available ELISA kits.

During admission, clinical information about the patients was collected, regarding demographic factors (age, gender), medical history (comorbidities, functional status) and related to the trauma (mechanism, severity-using GCS, single head or polytrauma). In addition, the presence of brain damage (intracranial lesion) was assessed by brain CT scan, together with its cause and extent. Scans were blindly read by an experienced neuroradiologist.

Three months after the event, a visit was performed, to assess the functional outcome of the patient using the extended Glasgow Outcome Score (GOSe). Whenever possible, this visit was scheduled with routine, clinically indicated, medical visits or as a telephonic visit as an alternative.

Healthy volunteers were enrolled among patients' relatives (control, n= 17).

### DATA PROTECTION

Samples and clinical information were coded with a number assigned in the electronic case registration form (CRF) with exclusive access to the research team. The remaining serum and plasma samples were stored in Neurovascular Research Laboratory at Vall d'Hebrón Institute of Research for later use, frozen at -80° C. Patients were informed about the possibility of these samples to be used in subsequent studies, within the research line of brain damage biomarkers in acute neurological conditions, in research projects approved by the Ethics Committee of Hospital Vall d'Hebrón.

### STATISTICAL ANALYSIS

Best cut-off (or reference value) to predict the absence of brain lesions on CT scan was calculated with ROC curves, by fixing a 100% sensitivity.

Additionally, the predictive value of the biomarkers was further evaluated. With the biomarkers that demonstrate a significant association (or trend towards it) with the presence of brain lesions (p <0.1), a logistic regression model was developed in which the biomarkers were the independent variables, being the dependent variable the presence of lesions on brain CT scan (which were classified as normal or pathological). Additionally, the predictive value of the biomarker panel and the best cut points were calculated with the PanelomiX software (https://www.panelomix.net/).

### RESULTS

Next tables 1A and 1B show the cut-off values for the selection of a patient suffering TBI that will give a positive CT scan in terms of displaying brain damage in closed-head injury. The cut-offs were selected for a sensitivity of 95-100%. The results derive from a cohort of 46 patients, of which 39 gave a negative CT scan and 7 gave a positive CT scan:

**Table 1A. Specificity values for individual markers and a sensitivity of 95-100%**

| Biomarker | Threshold (Cut-off) | Specificity | Sensitivity |
|---|---|---|---|
| A-FABP | 27.97 ng/ml | 23.08 | 100 |
| AQP4 | 0.82 ng/ml | 30.77 | |
| BCAN | 262.03 ng/ml | 41.03 | |
| FGFR1 | 115.63 ng/ml | 48.72 | |
| H-FABP | 10.30 ng/ml | 58.97 | |
| GFAP | 224.9 pg/ml | 43.59 | |
| IL-10 | 0.33 ng/ml | 30.77 | |
| S100B | 52.79 ng/ml | 20.51 | |

**Table 1 B_Specificity of panels of 2 proteins with a sensitivity set at 0.95-1**

| | A-FABP | AQP4 | BCAN | FGFR1 |
|---|---|---|---|---|
| A-FABP | - | | | |
| AQP4 | 46.2 | - | | |
| BCAN | 53.8 | 56.4 | - | |
| FGFR1 | **61.5** | **64.1** | 53.8 | - |
| H-FABP | 59 | **69.2** | **69.2** | **74.4** |
| GFAP | 48.7 | 59 | **64.1** | **71.8** |
| IL-10 | 41 | 53.8 | 56.4 | 64.1 |
| S100B | 30.8 | 41 | 51.3 | 59 |

FGFR1 proved individually a high specificity to rule out mTBI with no need to perform CT scans. The combination of the levels of FGFR1 with other markers related with TBI screening highly improved its specificity.

Combining the levels of H-FABP with AQP4 or with BCAN or with FGFR1, specificity values were increased to around 70 % and up to 74 %. Similar specificities were observed for the combination of any of AQP4 or with BCAN or with FGFR1 with GFAP (specificities values of 60-72%).

Next Tables 2A and 2B illustrated the outcome prediction capability of the tested proteins, individually and in combination of panels of 2 proteins. The cut-offs were selected for a sensitivity of 95-100%. The results derive from a cohort of 34 patients, classified as GOSE=8 (complete recovery) or as GOSE<8 (incomplete recovery):

**Table 2A. Specificity values for individual markers and a sensitivity of 95-100%**

| Biomarker | Threshold (Cut-off) | Specificity | Sensitivity |
|---|---|---|---|
| A-FABP | 27.96 ng/ml | 20.69 | 100 |
| AQP4 | 1.26 ng/ml | 48.26 | |
| BCAN | 253.3 ng/ml | 48.28 | |
| FGFR1 | 136.53 ng/ml | 37.93 | |
| H-FABP | 19.09 ng/ml | 58.97 | |
| GFAP | 224.9 pg/ml | 55.17 | |
| IL-10 | 204.30 ng/ml | 27.58 | |
| S100B | 72.02 ng/ml | 31.03 | |

**Table 2B_Specificity of panels of 2 proteins with a sensitivity set at 0.95-1**

| | A-FABP | AQP4 | BCAN | FGFR1 |
|---|---|---|---|---|
| A-FABP | - | | | |
| AQP4 | **69** | - | | |
| BCAN | 58.86 | **75.9** | - | |
| FGFR1 | 48.3 | **72.4** | 51.7 | - |
| H-FABP | 55.2 | **69** | **69** | **65.5** |
| GFAP | **75.9** | 58.6 | 58.6 | 51.7 |
| IL-10 | 58.6 | **75.9** | **69** | **62.1** |
| S100B | 37.9 | **72.4** | 58.6 | 51.7 |

Combining AQP4 levels with either H-FABP, GFAP, IL-10 or S100B improved considerably the specificity of the assay.

Combining AQP4 with BCAN or FGFR1, gives high specificities (>70%).

Combining A-FABP with GFAP, gives high specificities around 76%.

With the methods of the invention and as derivable from data in the examples, a patient with symptoms of TBI, in particular mild TBI can be first assessed using for example the Glasgow Coma Scale. Then the levels of the proteins indicated in this invention can be determined in an isolated sample in order to rule out a CT scan. If the markers indicate that a CT scan can be ruled out, the patient is safely sent at home. On the contrary, if according to the in vitro methods of the invention the patients is classified as having intracranial lesions (although not apparent), then a CT scan is performed to finally decide if the patient stays in hospital (positive CT scan with observable lesions), or if the patient is finally discharged (negative CT scan with no observable lesions).

Thus, the methods, systems and kits of the invention suppose a real contribution to the Health System since they suppose reliable and cost-effective routes of correctly classifying a potential mTBI patient.

### Citation List

- Narayan, R. et al. 2002. Clinical trials in head injury. Journal of Neurotrauma. Vol. 19(5). pp:503-57. doi: 10.1089/089771502753754037.
- Burtis C. A. et al., 2008, Chapter 14, section "Statistical Treatment of Reference Values"
- Jennett, B; Bond, M (Mar 1, 1975). "Assessment of outcome after severe brain damage". Lancet. 1 (7905): 480-484.
- BLASTManual, Altschul, S., et al, NCBI NLM NIH Bethesda, Md. 20894, Altschul, S., et al, J. Mol. Biol. 215: 403-410 (1990)

## Claims

1. An *in vitro* method for selecting a patient suffering traumatic brain injury (TBI) for a brain image technique and/or for a subsequent treatment intervention, comprising determining the level of one or more of the proteins selected from the group consisting of fibroblast growth factor receptor 1 (FGFR1), Aquaporin-4 (AQP4), Brevican Core Protein (BCAN) and Fatty acid-binding protein in adipocytes (A-FABP), in an isolated biofluid sample of said patient.

2. The *in vitro* method for selecting a patient suffering traumatic brain injury (TBI) according to claim 1, wherein the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

3. The *in vitro* method for selecting a patient suffering traumatic brain injury (TBI) according to any one of claims 1-2, comprising determining the level of expression of at least one set of proteins selected from the group consisting of: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B; BCAN and FGFR1; BCAN and H-FABP; BCAN and IL-10; BCAN and S100B; FGFR1 and H-FABP; FGFR1 and GFAP; FGFR1 and IL-10; and FGFR1 and S100B.

4. The in vitro method for selecting a patient suffering traumatic brain injury (TBI) according to any one of claims 1-3, wherein the level of the one or more proteins are compared with a corresponding reference value and the patient is selected for a brain image technique and/or for a subsequent treatment intervention when the level of FGFR1 and/or of BCAN is lower than a reference value; and/or when the level of A-FABP and/or of AQP4 is higher than a reference value.

5. The in vitro method for selecting a patient suffering traumatic brain injury (TBI) according to any one of claims 1-4, wherein the brain image technique is tomography computer tomography (CT) and/or magnetic resonance imaging (MRI); and the treatment intervention is selected from surgery for relieving pressure in the skull, and/or surgery for removing clotted blood and/or surgery for repairing skull fractures, medication with neuroprotectants, and/or medication with anticoagulants and/or medication with anticonvulsants, and/or physical and/or psycological rehabilitation.

6. An in vitro method for screening for TBI patients with intracranial lesion, comprising determining the level of one or more of the proteins selected from the group consisting of FGFR1, AQP4, BCAN and A-FABP in an isolated biofluid sample of said patient.

7. The in vitro method for screening for TBI patients with intracranial lesion according to claim 6, wherein the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

8. The in vitro method for screening for TBI patients with intracranial lesion according to any one of claims 6-7, comprising determining the level of expression of at least one set of proteins selected from the group consisting of: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B;BCAN and FGFR1; BCAN and H-FABP; BCAN and IL-10; BCAN and S100B; FGFR1 and H-FABP; FGFR1 and GFAP; FGFR1 and IL-10; and FGFR1 and S100B.

9. The in vitro method for screening for TBI patients with intracranial lesion according to any one of claims 6-8, wherein the level of the one or more proteins are compared with a corresponding reference value and the patient is determined as having intracranial lesion when the level of FGFR1 and/or of BCAN is lower than a reference value; and/or when the level of A-FABP and/or of AQP4 is higher than a reference value.

10. The *in vitro* method for selecting a patient suffering traumatic brain injury (TBI) according to any one of claims 1-5, or for screening for TBI patients with intracranial lesion according to any one of claims 6-9, wherein the biofluid sample is selected from the group consisting of blood, serum, plasma, urine, saliva, lachrymal fluid, cerebrospinal fluid, and combinations thereof.

11. The *in vitro* method for selecting a patient suffering traumatic brain injury (TBI) according to any one of claims 1-5, or for screening for TBI patients with intracranial lesion according to any one of claims 6-9, wherein traumatic brain injury is mild traumatic brain injury (mTBI).

12. An in vitro prospective method for determining a recovery outcome in an isolated biofluid sample of a subject having experienced traumatic brain injury (TBI), comprising determining the level of one or more of the proteins selected from the group consisting of FGFR1, AQP4, BCAN and A-FABP in an isolated biofluid sample of said patient.

13. An in vitro prospective method according to claim 12, wherein the levels of at least two different proteins are determined, being one of the at least two proteins selected from FGFR1, AQP4, BCAN and A-FABP, and the second of the at least two proteins selected from FGFR1, AQP4, BCAN, A-FABP, H-FABP, GFAP, IL-10, and S100B.

14. An in vitro prospective method according to any one of claims 12-13, comprising determining the level of expression of at least one set of proteins selected from the group consisting of: A-FABP and AQP4; A-FABP and BCAN; A-FABP and FGFR1; A-FABP and H-FABP; A-FABP and GFAP; A-FABP and IL-10; A-FABP and S100B; AQP4 and BCAN; AQP4 and FGFR1; AQP4 and H-FABP; AQP4 and GFAP; AQP4 and IL-10; and AQP4 and S100B; BCAN and FGFR1; BCAN and H-FABP; BCAN and IL-10; BCAN and S100B; FGFR1 and H-FABP; FGFR1 and GFAP; FGFR1 and IL-10; and FGFR1 and S100B.

15. A kit comprising reagent means for detecting the level of two or more of the proteins selected from FGFR1, AQP4, BCAN and A-FABP.
